# EUROPÄISCHE PATENTSCHRIFT

(11) **EP 0 682 511 B1**
(45) Veröffentlichungstag und Bekanntmachung des Hinweises auf die Patenterteilung: **22.10.1997**
(21) Anmeldenummer: 94905674.1
(22) Anmeldetag: 21.01.1994
(51) Int. Cl.: A61K 6/033

(54) **KERAMISCHES MATERIAL FÜR ZAHNFÜLLUNGEN UND/ODER ZAHNERSATZ UND VERFAHREN ZUR HERSTELLUNG DESSELBEN**
CERAMIC MATERIAL FOR TOOTH FILLINGS AND/OR PROSTHESES AND PROCESS FOR PRODUCING THE SAME
MATERIAU CERAMIQUE POUR OBTURER DES DENTS ET/OU POUR REALISER DES PROTHESES ET SON PROCEDE DE PRODUCTION

(30) Priorität: 26.01.1993 DE 4302072
(43) Veröffentlichungstag der Anmeldung: 22.11.1995
(73) Patentinhaber: WIEDEMANN, Wolfgang, D-97204 Höchberg (DE)
(72) Erfinder: GUNDLACH, Hans-Werner, D-28209 Bremen (DE); WIEDEMANN, Wolfgang, D-97204 Höchberg (DE)
(74) Vertreter: Pöhner, Wilfried Anton, Dr.
(86) Internationale Anmeldenummer: EP9400158
(87) Internationale Veröffentlichungsnummer: WO9416666

(56) Entgegenhaltungen:
- WO-A-89/05625
- DE-A- 3 831 260
- DE-A- 3 935 060
- US-A- 4 518 430
- JOURNAL OF DENTAL RESEARCH Bd. 50, Nr. 4 , 1971 Seiten 860 - 861 E. A. MONROE E.A. 'New Calcium Phosphate Ceramic Material for Bone and Tooth Implants'
- *Explosivverdichtung pulvriger Substanzen, R.Prümmer Springer-Verlag 1987

## Beschreibung

Die Erfindung betrifft ein Verfahren zur Herstellung eines keramischen Materials für Zahnfüllung und/oder Zahnersatz auf der Basis von Calciumphosphtverbindungen mit mindestens zwei unterschiedlichen Phasen oder Kristallarten aus einer schwerlöslichen Calciumphosphatverbindung und einer leichter lösbaren Substanz oder Verbindung, wobei ein wässriges Fällungsprodukt hergestellt, getrocknet und gesintert wird.

Zur Beseitigung insbesondere kariöser Defekte im Zahnschmelz und zur Herstellung von Zahnersatz, wie zum Beispiel Inlays, Kronen und Brücken, sind unterschiedliche Materialien bekannt. Verbreitet sind Amalgame, Kunststoffe und Edelmetalle (zum Beispiel Gold). Jedes dieser Materialien hat spezifische Nachteile. Es können Allergien auftreten (Amalgam), die mechanischen Eigenschaften sind nicht ausreichend (Kunststoffe) oder die Kosten sind ungewöhnlich hoch (Gold).

Um die vorstehend genannten Nachteile auszuschalten, ist man bemüht, ein Material zu finden, das dem natürlichen Zahnschmelz möglichst ähnlich ist. Es sind deshalb Calciumphosphatverbindungen, insbesondere Hydroxylapatite, entwickelt worden. Diese kommen hinsichtlich der Farbe und der machanischen Eigenschaften dem natürlichen Zahnschmelz recht nahe. Sie haben aber die nachteilige Eigenschaft, daß ihre Oberfläche mit der Zeit rauh wird. Mit zunehmender Zerklüftung der Oberfläche wird das Hydroxylapatit schneller abgetragen (Deminaralisation). Zur Vermeidung oder Verzögerung dieses Abtrags sind sogenannte bioaktive Hydroxylapatite entwickelt worden, die eine die Demineralisation weitestgehend rückgängig machende Remineralisationseigenschaft aufweisen und beispielsweise in den Druckschriften DE 39 35 060 A1 oder DE 38 31 260 A1 beschrieben sind. Sie bestehen aus einer schwer löslichen Calciumphosphatverbindung und einer leichter löslichen Substanz und werden durch wässrige Fällung mit nachfolgender Trocknung und Sinterung hergestellt. Jedoch sind solche Materalien über das Laborstadium nicht hinausgekommen, da sie spezifische Parameter aufweisen müssen, die eine Massenherstellung mangels ausreichender Reproduzierbarkeit nicht zulassen.

Hiervon ausgehend liegt der Erfindung die Aufgabe zugrunde, ein Verfahren zu schaffen, welches die einfache und zuverlässige Herstellung eines keramischen Materials für Zahnfüllungen und Zahnersatz ermöglicht, das ein zuverlässiges Remineralisationsverhalten zeigt.

Diese Aufgabe wird erfindungsgemäß dadurch gelöst, daß das Fällunsprodukt nach dem Trocknen explosionsartig verdichtet wird.

Durch das erfindungsgemäße erxplosionsartige Verdichten des getrockneten und körnigen Fällungsprodukts vor dem anschließenden Sintern wird ein sogenannter Grünkörper geschaffen, der nach dem Sintern ein besonders günstiges De- und Remineralisierungsverhalten zeigt. Besonders gute Ergebnisse zeigt das explosionsartige Verdichten des feinkörnigen Fällungsprodukts zur Herstellung der nachfolgend beschriebenen keramischen Materialien.

Durch die Bildung des keramischen Materials aus getrennten Phasen oder Kristallen unterschiedlicher Materialien einerseits und die Bildung einer Phase bzw. eines Kristalls aus einem leichter löslichem Stoff erfolgt eine gezielte Demineralisation. Es werden im wesentlichen gleichmäßig gerasterte Hohlräume in den die eingentliche Zahnfüllung oder den eigentlichen Zahnersatz bildenden Calciumphosphatverbindungen gebildet. Die Calciumphosphatverbindungen erhalten so eine gezielte Porösität. Die Größe der Poren und der Anteil der Poren am gesamten keramischen Material kann durch die jeweilige leichter lösliche Substanz oder Verbindung gesteuert werden. Es entsteht so ein Gerüst, das hinsichtlich seines Aufbaus und seiner Freiräume gezielt vorausbestimmbar ist. Dadurch lassen sich Bedingungen im keramischen Material schaffen, die eine optimale Remineralisation gewährleisten.

Als besonders vorteilhaft hat es sich erwiesen, ein unstöchiometrisches Verhältnis der einzelnen Phasen oder Kristalle zu wählen.

Vorzugsweise handelt es sich bei den leichter löslichen Substanzen oder Verbindungen um solche, die durch Säure löslich sind. Das Herauslösen der entsprechenden Kristalle kann so gezielt gesteuert werden durch die Herbeiführung saurer Verhältnisse im Munde, insbesondere im Speichel. Das kann gefördert werden dadurch, daß der Patient einen sauren pH-Wert aufweisende oder einen sauren pH-Wert im Munde erzeugende Solvetten lutscht.

Bevorzugt handelt es sich bei der leichter löslichen Substanz oder Verbindung um Alpha- und/oder Beta-Triclciumphosphat (Whitlockit) oder ein Gemisch aus beiden. Dieses läßt sich bei der Herstellung der anderen Phase, nämlich der Calciumphosphatverbindung, leicht bilden und in Form von gesonderten Kristallen gleichmäßig verteilt im keramischen Material unterbringen.

Vorzugsweise werden Kristalle aus Alpha- und/oder Beta-Tricalciumphosphat in die Kristalle und/oder Zwischenräume zwischen benachbarten Kristallen der Calciumphosphatverbindung eingelagert. Das Alpha- und/oder Beta-Tricalciumphosphat bildet so "Platzhalter" im erfindungsgemäßen keramischen Material, die nach dem Herauslösen des Alpha- und/oder Beta-Tricalciumphosphats zur gewünschten Porösität führen.

Das keramische Material verfügt nach einer Weiterbildung der Erfindung über ein Calcium-Dezifit. Vorzugsweise wird dieses herbeigeführt durch ein Atomverhältnis von Calcium zu Phosphor, das kleiner als 1,666 ist, insbesondere im Bereich zwischen 1,580 und 1,665 liegt. Auch hierdurch wird wirksam eine Mischkristallbildung vermieden.

Ein besonders günstiges Remineralisationsverhalten des keramischen Materials stellt sich ein, wenn der Anteil des Alpha- und/oder Beta-Tricalciumphosphats am Gesamtgewichtsanteil des keramischen Materials 1 % bis 50 %, insbesondere 2 % bis 30 % beträgt. Vorzugsweise wird zusätzlich angestrebt ein Alpha- und/oder Beta-Tricalciumphosphat und Hydroxylapatit mit einer Kristallgröße von 0,1 µm bis 10 µm, und zwar insbesondere 0,5 µm bis 5 µm.

Vorzugsweise wird das explosionsartige Verdichten isostatisch durchgeführt. Dadurch erhält das hergestellte Material besonders gleichmäßige Eigenschaften. Alternativ ist es so auch denkbar, das explosionsartige Verdichten ein- oder mehrachsig durchzuführen.

Nach einem weiteren Aspekt der Erfindung wird eine unstöchiometrische Calciumverbindung (insbesondere Hydroxylapatit) verwendet. Diese führt beim Sintern zur Bildung unterschiedlicher, nämlich leichter und schwerer löslicher, Phasen bzw. Kristalle.

Nach einem weiteren Vorschlag der Erfindung wird das Fällungsprodukt vor dem Trocknen mindestens einmal gewaschen. Dadurch werden unerwünschte Bestandteile einfach und zuverlässig beseitigt.

Nach der Erfindung findet das Trocknen des Fällungsmaterials bei etwa 200°C statt. Ein solches Trocknen läßt sich schonend durchführen, ohne daß negative Einflüsse auf die späteren Eigenschaften des keramischen Materials zu befürchten sind. Nach dem Trocknen erfolgt gemäß einer bevorzugten Weiterbildung des Verfahrens eine Reifung bzw. Nachprüfung der Kristallite des festen und/oder gemahlenen Fällungsprodukts mit einem gespannten Fluid, insbesondere Dampf.

Nachfolgend wird die Erfindung anhand bevorzugter Ausführungsbeispiele näher erläutert:

Das erfindungsgemäße keramische Material kommt im festen Zustand im dentalen Bereich zur Anwendung für Zahnrestaurationen und Zahnersatz, wie Inlays, Kronen und Brücken. Dazu wird aus einem Rohling des keramischen Materials vorzugsweise durch computergesteuertes Fräsen ein entsprechendes (Einsatz-)Teil hergestellt.

Das feste keramische Material besteht aus isolierten Kristallen unterschiedlicher Substanzen, und zwar aus Kristallen einer schwerer löslichen Substanz und darin eingelagerte Kristalle einer leichter löslichen Substanz. Die schwerlösliche Substanz besteht im wesentlichen aus Fluorapatit oder Hydroxylapatit mit vorzugsweise bis zu 2 % Carbonationen im Gitter. Es kann modifiziert sein durch Anteile von Fluorapatit, geringe Mengen amorpher, glasartiger oder polykristalliner Bestandteile von Hydrogenphosphaten, Pyrophosphaten und/oder Polyphosphaten.

Die leichter lösliche Substanz besteht aus Alpha-Tricalciumphosphat oder Beta-Tricalciumphosphat. Alternativ kann die leichter lösliche Substanz auch aus Mischungen von Alpha- und Beta-Tricalciumphosphat gebildet sein. Es kann zur Bildung der leichter löslichen Substanz reines Alpha- und/oder Beta- Tricalciumphosphat verwendet werden, aber auch Mischungen aus Hydroxylapatit mit Alpha- oder Beta-Tricalciumphosphat oder Hydroxylapatit mit sowohl Alpha- als auch Beta-Tricalciumphosphat.

Überraschend gute Beständigkeit hat ein keramisches Material, bei dem der Gewichtsanteil der zweiten, leichter löslichen Phase aus Alpha- und/oder Beta-Tricalciumphosphat 1 % bis 50 %, insbesondere 2 % bis 30 % beträgt. Die Kristallgröße ist vorzugsweise bei beiden Phasen, also den leichter und den schwerer löslichen Substanzen, etwa gleich groß, beträgt nämlich zwischen 0,1 µm und 10 µm, insbesondere 0,5 µm bis 5 µm. Bei den Kristallen handelt es sich vorzugsweise um isometrische Kristallite.

Während reiner Hydroxylapatit ein Atomverhältnis von Calcium zu Phosphor aufweist, das 1,666 beträgt, weist das erfindungsgemäße keramische Material ein Calcium-Defizit auf. Das Calcium/Phosphor-Atomverhältnis beträgt insbesondere 1,580 bis 1,665.

Ein keramisches Zahnfüllungsmaterial, ein Dentalzement oder ein Dentalkleber, die zur Verarbeitung flüssig bzw. pastös sind, lassen sich ebenfalls unter Verwendung des vorstehend erwähnten keramischen Materials herstellen. Das keramische Material dient hierbei als Grundlage für einen feinkörnigen Füllstoff. Dazu wird das fertige, harte Material gemahlen. Es wird sodann vermischt mit einem Kunststoff. Vorzugsweise handelt es sich dabei um ein zumindest teilweise resorbierbaren Kunststoff. Der Kunststoff wird mit einer gleichen oder größeren Menge des pulverisierten keramischen Materials vermischt. Er liegt dann als Paste oder Kleber vor. Das pastöse Zahnfüllmaterial wird in die Kavität eingebracht und härtet nach dem Einbringen aus, und zwar gegebenenfalls unter Beaufschlagung mit Energie, insbesondere Licht.

Alternativ ist es auch möglich, mit Füllstoffen aus dem keramischen Material angereicherte resorbierbare Kunststoffe aushärten zu lassen zu festen Körpern. Diese können als Halbzeuge dienen für die Herstellung von Zahnersatz durch beispielsweise Fräsen.

Die Herstellung des erfindungsgemäßen keramischen Materials erfolgt zunächst so, daß Calciumverbindungen, wie zum Beispiel Calciumnitrat, mit Phosphorverbindungen (Ammoniumhydrogenphosphat) in einer alkalisierten, wässrigen Base, wie zum Beispiel Natriumhydroxid, Kaliumhydroxid oder Ammoniumhydroxid, ausgefällt werden. Als primäres Fällungsprodukt entsteht nichtstöchiometrischer Hydroxylapatit. Bei entsprechender Reaktionstemperatur, Konzentration der eingesetzten Komponenten und Reaktionszeit wachsen primär ausgefällte, unperfekte Hydroxylapatit-Kristallite zu mehr oder minder stöchiometrischem Hydroxylapatit. Beispielsweise kann das Fällungsprodukt auch nach dem aus der DE-OS 39 35 060 bekannten Verfahren hergestellt werden.

Für die Erfindung von wesentlicher Bedeutung ist die Reihenfolge der Zugabe der einzelnen Substanzen. Die Lösung von Ammoniumhydrogenphosphat wird demnach langsam in eine Vorlage eingegeben. Die Vorlage enthält die gesamte Reaktionsmenge an Calciumnitrat. Dabei bildet sich vorzugsweise Hydroxylapatit.

Des weiteren wird das Fällungsprodukt mit destilliertem Wasser vorzugsweise mehrfach gewaschen. Bevorzugt findet heißes destilliertes Wasser Anwendung. Anschließend erfolgt das Trocknen. Das trockene, feste Fällungsprodukt wird nunmehr durch Mahlen oder Mörsern zerkleinert. Vorzugsweise wird das Fällungsprodukt pulverisiert.

Anschließend erfolgt in gespanntem Wasserdampf bei Temperaturen zwischen 100°C und 350°C während 1 Stunde bis zu 10 Tagen eine Nachreifung der Kristallite.

Nach einem wesentlichen Merkmal der Erfindung wird das pulverisierte Fällungsprodukt durch explosionsartiges Zusammenpressen verdichtet zur Bildung eines Grünkörpers. Daraus wird dann das fertige (feste) keramische Material bei Temperaturen von 1000°C bis 1300°C über einen Zeitraum von 15 Minuten bis 24 Stunden gesintert.

Obwohl das nach dem erfindungsgemäßen Verfahren gebildete keramische Material im Aussehen und in seinen physikalischen Eigenschaften weitgehend dem natürlichen Zahnschmelz entspricht, nämlich weißlich transparent ist, können dem Material Pigmente zugegeben werden zur Erzielung der Farben individueller Zähne.

Es ist auch möglich, das erfindungsgemäße keramische Material vor der Weiterverarbeitung, insbesondere vor der Einbringung in den Mund des Patienten, einer Säurebehandlung zu unterziehen, so daß das leichtlösliche Alpha- und/oder Beta-Tricalciumphosphat schon aus dem Hydroxylapatit herausgelöst wird, bevor das nur noch aus Hydroxylapatit bestehende keramische Material in den Mund des Patienten eingesetzt wird. Hier braucht dann nur noch durch das Hineindiffundieren von calciumphosphathaltigem Speichel neu abgeschiedener Hydroxylapatit gebildet zu werden zum Wiederauffüllen der durch das Auslösen von Alpha- und/oder Beta-Tricalciumphosphat entstandenen Poren im keramischen Material.

## Patentansprüche

1. Verfahren zur Herstellung eines keramischen Materials für Zahnfüllung und/oder Zahnersatz auf der Basis von Calciumphosphtverbindungen mit mindestens zwei unterschiedlichen Phasen oder Kristallarten aus einer schwerlöslichen Calciumphosphatverbindung und einer leichter lösbaren Substanz oder Verbindung, wobei ein wässriges Fällungsprodukt hergestellt, getrocknet und gesintert wird, **dadurch gekennzeichnet,** daß das Fällunsprodukt nach dem Trocknen explosionsartig verdichtet wird.

2. Verfahren nach Anspruch 1, **dadurch gekennzeichnet**, daß das explosionsartige Verdichten des Fällungsproduktes isostatisch durchgeführt wird.

3. Verfahren nach Anspruch 1 oder 2, **dadurch gekennzeichnet,** daß das Fällungsprodukt vor dem Trocknen mindestens einmal gewaschen wird, vorzugsweise mit heißem destilliertem Wasser.

4. Verfahren nach einem der Ansprüche 1 bis 3, **dadurch gekennzeichnet**, daß das Fällungsprodukt in gespanntem Wasserdampf nachgereift wird.

5. Verfahren nach Anspruch 4, **dadurch gekennzeichnet**, daß das Fällungsprodukt in gespanntem Wasserdampf von 100°C bis 350°C während eines Zeitraums von 1 Stunde bis 10 Tagen nachgereift wird, wobei die Nachreifung vorzugsweise nach dem Trocken und Zerkleinern des Fällungsproduktes erfolgt.

6. Verfahren nach einem der Ansprüche 1 bis 5, **dadurch gekennzeichnet,** daß als wässriges Fällungsprodukt eine Calciumphosphatverbindung verwendet wird.

7. Verfahren nach einem der Ansprüche 1 bis 6, **dadurch gekennzeichnet,** daß eine nichtstöchiometrische Calciumphosphatverbindung verwendet wird.

## Claims

1. Process for producing a ceramic material for dental fillings and/or dentures based on calcium phosphate compounds having at least two different phases or crystal types from a slightly soluble calcium phosphate compound and more soluble substance or compound, whereby a hydrous precipitation product is produced, dried and sintered, **wherein** the precipitation product is compacted explosively after drying.

2. Process according to claim 1, **wherein** the explosive compacting of the precipitation product is executed isostatically.

3. Process according to claim 1 or 2, **wherein** the precipitation product is washed at least once before drying, preferably with hot distilled water.

4. Process according to one of claims 1 to 3, **wherein** the precipitation product is subsequently matured in pressurised water vapour.

5. Process according to claim 4, **wherein** the precipitation process is subsequently matured in pressurised water vapour from 100°C to 350°C during a period of 1 hour to 10 days, whereby the subsequent maturing is preferably executed after the drying and comminution of the precipitation product.

6. Process according to one of claims 1 to 5, **wherein** a calcium phosphate compound is used as a hydrous precipitation product.

7. Process according to one of claims 1 to 6, **wherein** a non-stoichiometric calcium phosphate compound is used.

## Revendications

1. Procédé pour la fabrication d'une céramique destinée à l'obturation dentaire et/ou les prothèses dentaires à base de composés de phosphate de calcium comportant au moins deux phases ou types de cristaux différents provenant d'une combinaison à faible solubilité de phosphate de calcium et d'une substance ou d'une combinaison plus facilement soluble, un précipité étant ainsi fabriqué, séché et fritté caractérisé par le fait que le précipité est compacté par explosion après séchage.

2. Procédé selon la revendication 1 caractérisé par le fait que le compactage par explosion a lieu de façon isostatique.

3. Procédé selon la revendication 1 ou 2 caractérisé en ce que le précipité est lavé au moins une fois avant le séchage, de préférence, en utilisant de l'eau distillée chaude.

4. Procédé selon l'une des revendications 1 à 3 caractérisé en que le précipité est stabilisé dans de la vapeur d'eau sous pression.

5. Procédé selon la revendication 4 caractérisé par le fait que le précipité est stabilisé dans de la vapeur d'eau sous pression de 100 à 350°C pendant une heure à 10 jours, la stabilisation ayant lieu de préférence après séchage et pulvérisation du précipité.

6. Procédé selon l'une des revendications 1 à 5 caractérisé par le fait que, comme produit de précipitation aqueuse, on utilise une combinaison de phosphate de calcium.

7. Procédé selon l'une des revendications 1 à 6 caractérisé par le fait qu'une combinaison de phosphate de calcium non-stoechiométrique est utilisée.
